# EUROPÄISCHE PATENTSCHRIFT

(11) **EP 0 391 330 B1**
(45) Veröffentlichungstag und Bekanntmachung des Hinweises auf die Patenterteilung: **01.03.1995**
(21) Anmeldenummer: 90106330.5
(22) Anmeldetag: 03.04.1990
(51) Int. Cl.: G01N 33/571, G01N 33/569, G01N 33/543

(54) **Verfahren zur Bestimmung von Antikörpern gegen Erreger von Infektionskrankheiten in Körperflüssigkeiten, Mittel dazu und ihre Verwendung in diesem Verfahren**
Method for the determination of antibodies against causative agents of infectious diseases, means therefore and its use
Procédé pour déterminer des anticorps contre des agents causant des maladies infectieuses, moyen pour ce procédé et son utilisation

(30) Priorität: 07.04.1989 DE 3911361
(43) Veröffentlichungstag der Anmeldung: 10.10.1990
(73) Patentinhaber: BEHRINGWERKE Aktiengesellschaft, 35001 Marburg (DE)
(72) Erfinder: Peters, Helmut, Dr., D-3550 Marburg (DE); Krupka, Udo, Dr., D-3550 Marburg (DE)

(56) Entgegenhaltungen:
- EP-A- 0 136 798
- EP-A- 0 264 866
- EP-A- 0 265 851
- EP-A- 0 294 535
- EP-A- 0 296 036
- JOURNAL OF VIROLOGICAL METHODS, vol. 19, 1988, Amsterdam (NL); Y. VARELA et al., Seiten 79-87#
- GENITOURIN MEDICINE, vol. 62, 1986, London (GB); R.V.W. VAN EIJK et al., Seiten 367-372#

## Beschreibung

Die Erfindung betrifft ein immunchemisches, kompetitives Verfahren zum Nachweis und zur Bestimmung von Antikörpern gegen Bakterien, Viren, Parasiten und Pilze. Mit einer Variante der beschriebenen Methode ist auch der Nachweis des Erregers in Körperflüssigkeiten möglich. Weiterhin werden Testbestecke zur Ausführung des Verfahrens und deren Anwendung beschrieben.

Eine besondere Ausführungsform erlaubt den quantitativen Nachweis von Antikörpern, insbesondere bei Syphilis.

In jüngster Zeit wurde die Bedeutung der Reaktivierung einer latenten Syphilis bei HIV-Patienten, welche häufig doppel-infiziert sind, erkannt. Besonders die Diagnose einer Neurosyphilis ist aufgrund der besonderen immunologischen Konstellation solcher Patienten schwierig. Die Nachweismethoden für Syphilis-Antikörper sind sehr vielfältig und reflektieren die frühzeitige diagnostische Beschäftigung (seit 1906) mit diesem Erreger, der noch Anfang dieses Jahrhunderts eine enorme gesundheitspolitische Bedeutung hatte.
Bei den Nachweismethoden für Syphilis-Antikörper können drei Kategorien unterschieden werden:
I. Nicht-Treponemen-spezifische Tests. Beispiele: Wassermann-Reaktion oder Cardiolipin-KBR, VDRL-Test oder Cardiolipin-Mikroflockungstest, RPR-Test: E.L. Reed, Public Health Lab. (1965) 23:96-103, VDRL-ELISA: N.S. Pedersen, O. Orum und S. Mouritsen, J. Clin.Microbiol. (1987) 25:1711-1716.
II. Treponemen-spezifische Tests, z.B. die Reiter-KBR oder der sogenannte Flagellum-ELISA (R.V.W. Van Eijk ,H.E. Menke, G.J. Tidemann und E. Stolz, Genitourin.Med. (1988) 62:367-372).
III. T. pallidum-spezifische Tests, z. B. der Nelson-Test (T.pallidum-Immobilisationstest), der "fluorescent treponemal antibody" (FTA)-Test (E.F. Hunter, W.E. Deacon und P.F. Meyer, Publ. Hlth.Rep. (1964) 79:410-412), der Treponema-pallidum-Hämagglutinationstest (TPHA: T. Tomizawa und S. Kasamatsu, Jap.J.med.Sci. Biol. (1966) 19:303-308).
   Indirekte Enzymimunoassays zum IgG- bzw. IgM-Nachweis beruhen auf der Bindung spezifischer Antikörper an T.pallidum-Antigenpräparationen, welche (in der Regel adsorptiv) an Festphasen gebunden sind. Die Detektion der gebundenen Antikörper erfolgt über enzym-markierte Zweitantikörper, gerichtet gegen humanes IgG bzw. IgM. Die Methode des indirekten ELISA zum Syphilis-IgM-Nachweis ist in US-Patent Nr. 4, 288, 426 beschrieben.

Zusammenfassend läßt sich sagen, daß keiner der in der Literatur beschriebenen und auf dem Markt befindlichen Tests die Anforderungen an einen Screening-Test hinsichtlich des vertretbaren technischen Aufwandes, der objektiven und quantitativen Testbewertung, der weitgehend automatisierbaren Durchführung und des erforderlichen Höchstmaßes an Sensitivität und Spezifität erfüllt.

Ein kompetitiver ELISA zum quantitativen Nachweis von Antikörpern gegen HIV ist in der europäischen Patentanmeldung EP-A-0 265 851 beschrieben.

Eine spezielle Ausführungsform des kompetitiven ELISA zur Quantifizierung von Rubella-Virus in Zellkultur ist bei Y. Varela, E. Ortega und B. Gomez, J.Virol.Meth. (1988) 19:79-87 beschrieben. Diese Autoren verwenden allerdings keine direkt-markierten Rubella-spezifischen Antikörper. WPI 86-130689/20 beschreibt die Anwendung des kompetitiven Testprinzips zum Nachweis von Rubella-Antikörpern.

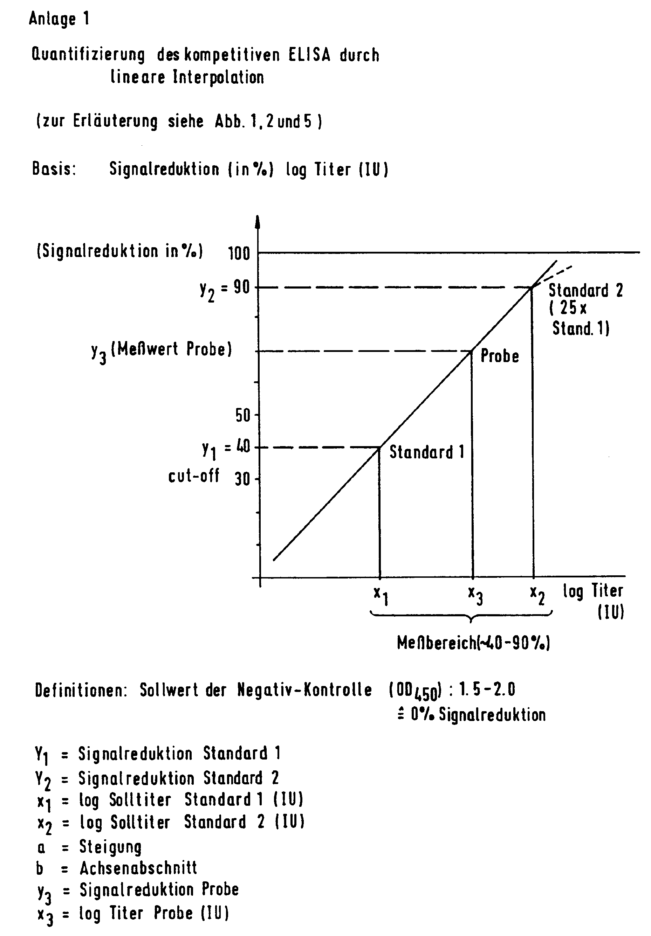

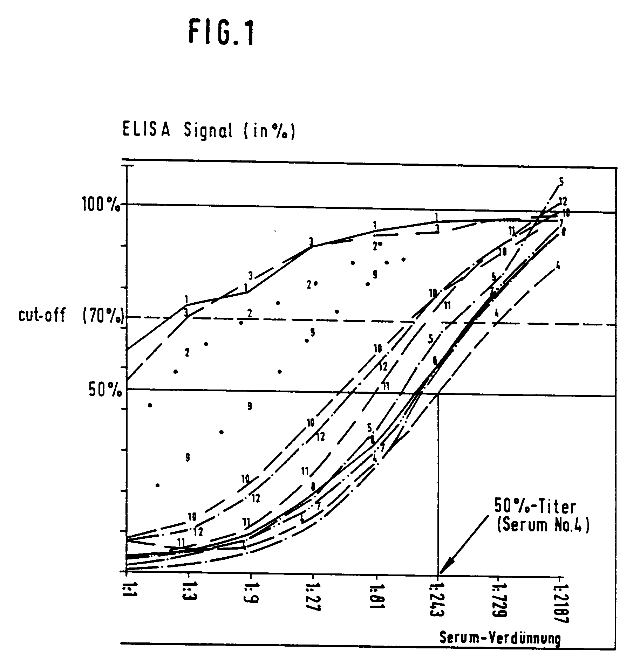

Die der Erfindung zugrundeliegende Aufgabenstellung bestand darin, einen hochspezifischen und hochsensitiven Nachweis für Antikörper gegen Bakterien, Viren und Parasiten zu entwickeln. Dieser Test sollte es erlauben, kleine und große Serien von Tests sowohl manuell als auch weitgehend automatisiert, z.B. mit Hilfe des Behring-ELISA-Processor II durchzuführen. Als Screening-Test sollte er, unter Berücksichtigung der geringen Prävalenz der Erkrankungen, vor allem eine sichere positiv/negativ-Diskriminierung ermöglichen. Hierbei ist es wesentlich, in einem solchen Test mit hoher Sicherheit auch das besonders infektiöse frühe Primärstadium mit in der Regel geringem IgG-Titer zu erfassen. Andererseits mußte die Spezifität des zu entwickelnden Testes hoch sein, um die Rate von falschpositiven Ergebnissen, die nur mit aufwendigeren Tests verifiziert werden können, möglichst klein zu halten. Es wurde nun gefunden, daß ein kompetitives Verfahren zum Nachweis und zur Bestimmung von Antikörpern gegen die Erreger von Syphilis, Toxoplasmose, Röteln, Cytomegalie, Amoebiasis, Echinokokkosis, Tetanus und Keuchhusten sowohl der IgM- als auch der IgG-Klasse möglich ist, bei Verwendung einer Festphase, an die die Antigene, die auch bis zu einem gewissen Grad verunreinigt sein können, direkt, also ohne Antikörper-vermittelte Bindung gebunden sind, wenn ein entsprechend ausgewählter Konjugatantikörper verwendet wird.
In Experimenten mit reinen, IgG-freien IgM-Präparationen konnte gezeigt werden, daß auch spezifische Igm-Antikörper das Meßsignal in signifikanter Weise reduzieren (Fig. 1; Proben 1, 2, 3).

Gegenstand der Erfindung ist ein immunchemisches, kompetitives Verfahren zum Nachweis und zur Bestimmung von Antikörpern gegen die Erreger von Syphilis, Toxoplasmose, Röteln, Cytomegalie, Amoebiasis, Echinokokkosis, Tetanus und Keuchhusten unter Verwendung einer Festphase bestehend aus einem Träger und daran irreversibel gebundenen Membranproteinen der jeweiligen Erreger und markierten Antikörpern, gerichtet gegen die wesentlichen Antigene des jeweiligen Erregers, wobei die Antikörper um die Bindungstelle an der Festphase konkurrieren, dadurch gekennzeichnet, daß die Proteine irreversibel direkt oder über einen nicht immunchemisch bindenden Spacer an den Träger gebunden sind.

Bevorzugt ist ein Verfahren zum Nachweis und zur Bestimmung von Antikörpern gegen die Erreger von Syphilis, Toxoplasmose und Röteln.

Gegenstand der Erfindung ist außerdem ein Testbesteck zum Nachweis und zur Bestimmung von Antikörpern in einem immunchemischen, kompetitiven Verfahren wie oben beschrieben, enthaltend einen Träger, an dem Membranproteine des jeweiligen Erregers direkt oder über einen nicht immunchemisch bindenden Spacer gebunden sind, markierte Antikörper gegen die genannten Membranproteine und gegebenenfalls Reagenzien zum Nachweis der Markierung.

Ferner ist Gegenstand der Erfindung die Verwendung des obengenannten Testbestecks in einem Verfahren zum Nachweis und zur Bestimmung von Antikörpern gegen die Erreger von Syphilis, Toxoplasmose, Röteln, Cytomegalie, Amoebiasis, Echinokokkosis, Tetanus und Keuchhusten.

Gegenstand der Erfindung ist auch ein Verfahren wie oben beschrieben, worin die Signalreduktion linear mit dem Logarithmus des Titers korreliert.

Gegenstand der Erfindung ist ferner ein Verfahren zum Nachweis und zur Bestimmung von Erregern von Syphilis, Toxoplasmose, Röteln, Cytomegalie, Amoebiasis, Echinokokkosis, Tetanus und Keuchhusten, wobei die Probe zunächst mit dem Konjugatantikörper in einem unbeschichteten Gefäß vorinkubiert wird und dann in das mit Antigenen des jeweiligen Erregers beschichtete Meßgefäß übertragen wird.

Die Titrationskurven von Syphilis-positiven Seren verlaufen über einen weiten Titerbereich linear und sind zueinander parallel (Fig. 1).

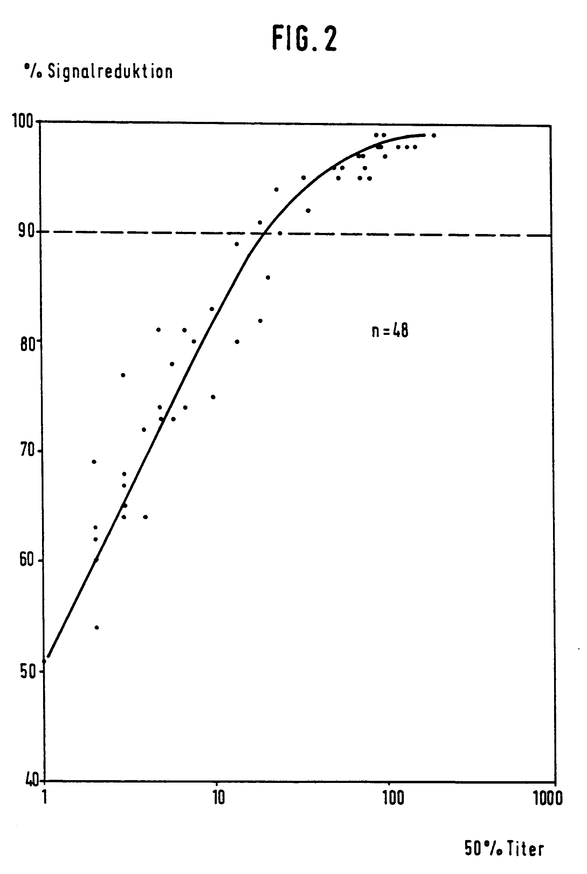

Diese Eigenschaft ist von Titrationskurven in indirekten ELISAs bekannt.
Tierische Hyperimmunsera zeigen eine vergleichbare Linearität und Parallelität und bieten sich als Basis für internationale Standards an. Zum quantitativen Vergleich des Antikörpergehaltes verschiedener Proben wurde z.B. der sogenannte 50%-Titer ermittelt, d.h. diejenige Serumverdünnung, bei welcher 50% des Signals der Negativ-Kontrolle erreicht werden (Fig. 1). Die Signalreduktion bei Testung der konzentrierten Serumprobe korreliert mit deren spezifischem Antikörpertiter.
Überraschenderweise zeigte es sich, daß bei Verwendung des erfindungsgemäßen Verfahrens die Signalreduktion (in % der Negativkontrolle) linear mit dem Logarithmus des Titers korreliert (Fig. 2). Diese Eigenschaft wird in einer speziellen Ausführungsform (siehe Beispiel 2) zur Quantifizierung der Syphilis-Antikörper herangezogen. Da nur solche Seren einen 50%-Titer aufweisen, die bei Testung der konzentrierten Proben das Meßsignal auf weniger als 50% der Negativkontrolle reduzieren, zeigt die Ordinate (Signalreduktion in % der Negativkontrolle) nur einen Meßbereich von 50 bis 100% Signalreduktion. In diesen Meßbereich fallen mehr als 95% der bisher untersuchten Proben.

Für Serumproben, die eine mehr als 90%-ige Signalreduktion aufweisen, gilt der lineare Zusammenhang zwischen der Signalreduktion und dem Logarithmus des Titers nicht mehr (siehe auch Fig. 1). Solche Proben müssen nach dem Screening-Test (mit unverdünntem Serum) nochmals nach Vorverdünnung (z.B. 1:20) nachgetestet werden.
Werden nun bei der quantitativen Ausführung der Technik mindestens 2 Standardseren (bzw. 2 Verdünnungsstufen eines Standardserums) mitgeführt, ist durch lineare Interpolation entsprechend Anlage 1 die Ermittlung des Titers bzw. des Antikörpergehaltes in internationalen Einheiten möglich. Diese lineare Interpolation kann auch rechnerisch, in der bevorzugten Ausführungsart der Auswertung auch mit Hilfe eines Computerprogrammes, durchgeführt werden.

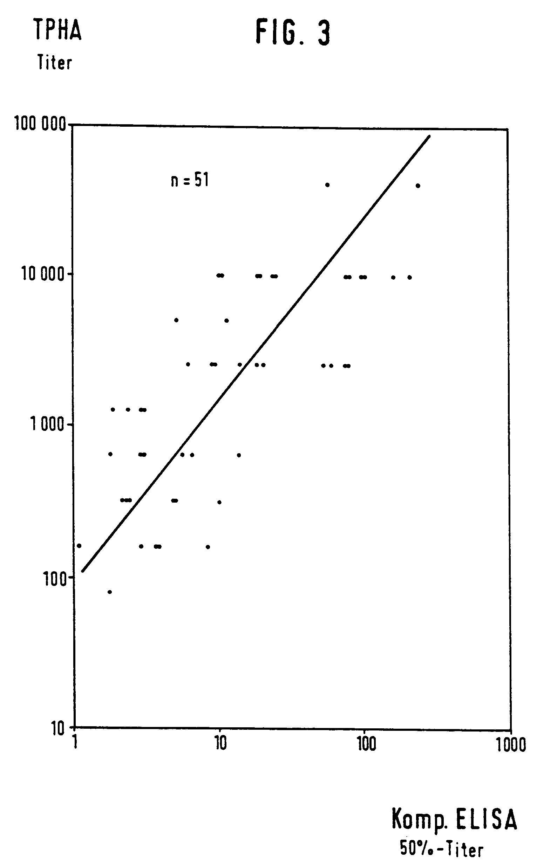

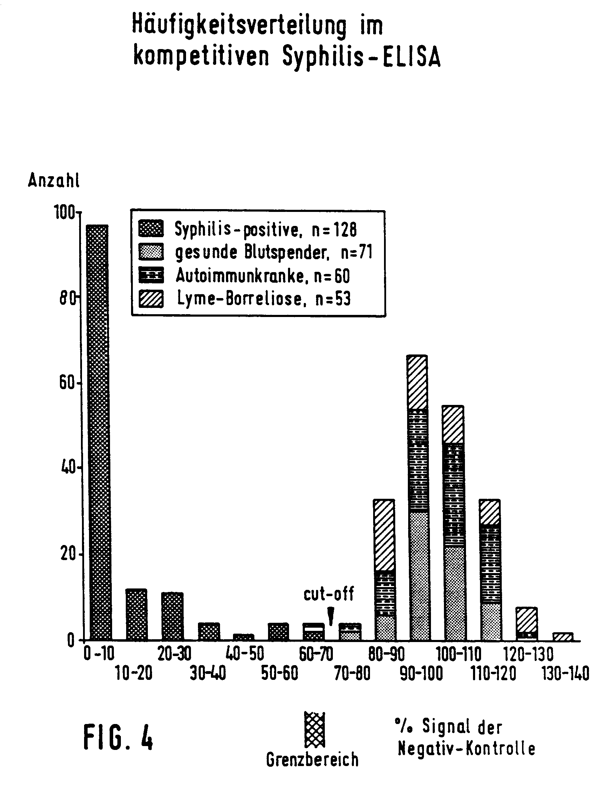

Weiterhin wurde beobachtet, daß bei Titration von Serumproben und Testung nach der beschriebenen Methode die Titer des kompetitiven ELISA mit denjenigen des TPHA linear korrelieren (Fig. 3).
Die hohe Sensitivität des Testes wurde durch Prüfung einer großen Zahl klinisch definierter Serumproben von Patienten aus allen Stadien der Syphilis (Fig. 4) belegt. Die hohe Spezifität wird belegt durch den negativen Reaktionsausfall von Proben von Autoimmunpatienten, von Borreliose-Patienten und einer großen Zahl von gesunden Blutspendern (Fig. 4).

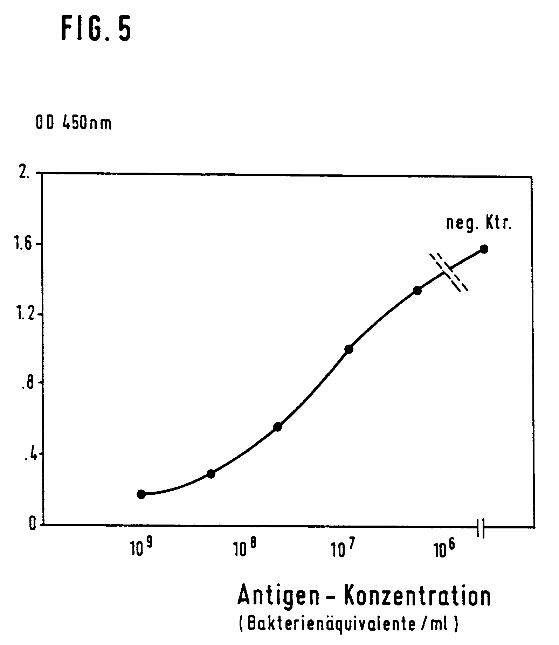

Überraschenderweise wurde weiterhin beobachtet, daß in einer besonderen Ausführungsform, nämlich durch Vorinkubation der Probe mit dem Konjugatantikörper in einem unbeschichteten Gefäß und anschließender Übertragung in das Gefäß, der Nachweis von Antigen möglich ist. Durch Versuche konnte verifiziert werden, daß losliche Bakterienäquivalente das Meßsignal in diesem Testansatz signifikant reduzieren (Fig. 5).

Die qualitative Version des kompetitiven ELISAs ermöglicht durch ihre einfache technische Durchführung vor allem, aber nicht ausschließlich, die Austestung von großen Probenmengen innerhalb von ca. 3 Stunden.
Aufgrund der zusätzlichen Erfassung von spezifischen IgM, auch in Abwesenheit von spezifischem IgG, ist eine sehr sensitive Detektion auch sehr früher Infektionsstadien möglich. In einer besonderen Ausführungsform, d.h. nach Vorinkubation der Probe mit dem POD-markierten Antikörper und anschließender Übertragung in das Meßgefäß, ist auch der Nachweis von Antigenen möglich. Die quantitative Version des kompetitiven ELISA liefert z.B. bei der Syphilis-Diagnostik für die Bewertung des Stadiums der Erkrankung bzw. der Bewertung des Erfolges einer Antibiotika-Therapie (neben den IgM-spezifischen Tests) wichtige Daten. Hier muß die gute Korrelation zum TPHA betont werden.
Die Machbarkeit dieses Prinzips konnte weiterhin belegt werden für den Nachweis von Antikörpern gegen Toxoplasma gondii und gegen das Rubella-Virus. Besondere Vorteile werden in der Tatsache gesehen, daß durch Auswahl des Konjugatantikörpers die Spezifität des Testes verbessert und aus diesem Grunde auf eine aufwendige Hochreinigung von Antigenen unter Umständen verzichtet werden kann.

Für die Festphase geeignete Antigenpräparationen können nach an sich bekannten Verfahren gewonnen werden z. B. Treponema pallidum nach WO 83/02886. Dabei werden die Treponemen aus den Hoden von infizierten Kaninchen gewonnen und mit dem nichtionischen Detergenz n-Octylglucosid extrahiert.

Als Trägermaterial für die Festphase sind Kunststoffe wie Polystyrol, Polyvinylchlorid, Polyamid und andere synthetische Polymere, natürliche Polymere wie Zellulose, sowie derivatisierte natürliche Polymere wie Zelluloseacetat und Nitrozellulose als auch Glas, insbesondere als Glasfaser geeignet.

Die Träger können die Form von Micropartikeln (0.01 bis 1 »m), Kugeln, Stäbchen, Röhrchen und Microtestplatten haben. Flächenförmige Gebilde wie Streifenpapiere, Plättchen und Membranen sind ebenfalls geeignet. Die Oberfläche der Träger kann sowohl für wässrige Lösungen durchlässig als auch undurchlässig sein.

Bevorzugte Träger sind Kugeln, Micropartikel, Röhrchen, Streifenpapiere und Membranen. Besonders bevorzugte Träger sind Microtestplatten.

Die Festphase wird hergestellt, indem eine Antigenpräparation irreversibel an den Träger gebunden wird.

Eine irreversible Bindung im Sinne der Erfindung liegt beispielsweise vor bei
1) einer adsorptiven Bindung, die nicht durch immunchemische Mittel, wie hochaffine Antikörper und auch nicht durch die in dem Verfahren verwendeten Mittel, wie markierte Antikörper, Verdunnungs- und Pufferlösungen gespalten wird,
2) einer über einen nicht immunchemisch bindenden Spacer vermittelte, bioaffine Bindung, wobei der Spacer aus Biotin und Avidin oder aus anderen Paarungen von Rezeptoren und Liganden bestehen kann,
3) einer kovalenten direkten Bindung
4) einer kovalenten, durch einen chemisch bifunktionellen Spacer vermittelten Bindung.

Bevorzugt wird die kovalente Bindung im Falle der Verwendung von wasserdurchlässigen Trägern sowie die adsorptive Bindung im Falle der Verwendung von wasserdurchlässigen als auch wasserundurchlässigen Trägern.

Besonders bevorzugt wird die direkte adsorptive Bindung von Antigenpräparationen an mit Gammastrahlen behandeltes Polystyrol als Träger.

Als Ausgangsmaterial für den Konjugat-Antikörper dient ein Poolserum von Spendern mit für den jeweiligen Erreger positiver Serologie. Monoklonale Antikörper sind ebenfalls geeignet.

Als Marker können verwendet werden radioaktive Isotope, fluoreszierende und chemilumineszierende Farbstoffe sowie Enzyme, deren Nachweis durch chromogene, luminogene oder fluorogene Substratsysteme möglich ist.

Die Markierung erfolgt nach Verfahren, die für die genannten Marker als Stand der Technik beschrieben sind.

Im Falle der Markierung der Antikörper mit Peroxidase kann die Periodat Technik nach Nakane et al., 1974, J. Histochem.Cytochem. 22, 1084-1090, angewandt werden oder ein Verfahren gemäß Ishikawa et al., 1983, J. Immunoassay 4, 209-327, in dem die Partner mit einem heterobifunktionellen Reagenz verknüpft werden.

Das erfindungsgemäße Verfahren kann beispielsweise durchgeführt werden, indem in die Kavitäten (Wells) einer Microtestplatte, die eine zuvor beschriebene Antigenpräparation gebunden enthält,
a) gleichzeitig die Probe und eine Lösung des markierten Antikörpers gegebenenfalls zuerst die Probe und nach einer gewissen Zeit eine Lösung des markierten Antikörpers gegeben, nach einer bestimmten Zeit der Inkubation die Lösung entfernt und die Wells mit einem Puffer gewaschen werden und anschließend die Markierung in den Wells gemessen wird,
   oder
b) zuerst eine Lösung enthaltend den markierten Antikörper gegeben wird, nach einer bestimmten Zeit der Inkubation die Lösung entfernt und die Wells mit einem Puffer gewaschen, die Microtestplatte gegebenenfalls getrocknet, anschließend die verdünnte Probe zugegeben und eine bestimmt Zeit inkubiert, dann die Probe aus den Wells entfernt und die Markierung in der Probe gemessen wird.

Das erfindungsgemäße Verfahren kann auch durchgeführt werden bei Verwendung eines diagnostischen Elements, das die Festphase und in trockener Form einen Teil oder auch alle erforderlichen Reagenzien enthält.

In einer bevorzugten Ausführung wird das erfindungsgemäße Verfahren zum Nachweis und zur Bestimmung von Erregern eingesetzt. Dabei wird beispielsweise zunächst in einer unbeschichteten Kavität einer Microtestplatte die Probe und eine Lösung des markierten Antikörpers inkubiert.

Nach einer bestimmten Zeit wird die vorinkubierte Lösung in eine mit Antigen beschichtete Kavität übertragen. Nach einer bestimmten Zeit der Inkubation wird die Lösung entfernt, die Kavität gegebenenfalls mit einem Puffer gewaschen und anschließend die Markierung in der Kavität gemessen.

Die folgenden Beispiele sollen die Erfindung erläutern und stellen keine Einschränkung auf die in den Beispielen genannten Ausführungsformen dar.

### Beispiele:

### 1. Bindung von T.palldium-Antigenen an Mikrotestplatten:

Treponema pallidum Spirochaeten werden aus Kaninchenhoden gewonnen, welche 10 bis 15 Tage vorher mit lebenden Erregern infiziert wurden. Die Treponemen werden durch Zentrifugation bei 48.000 x g mit 0,1 M Phosphatpuffer, pH 8,0 dreimal gewaschen, auf eine Keimzahl von 10⁹/ml eingestellt und wie in WO 83/02886 beschrieben, mit dem nichtionischen Detergenz n-Octylglucosid extrahiert.
Die solchermaßen gewonnene Antigenlösung mit einem Proteingehalt von > 0,2 mg/ml wird in Verdünnungsstufen von 1:50, 100, 150, 200, 300, 400, 600 und 800 verdünnt. Als Testplatte werden teilbare Polystyrol-Mikrotitrationsplatten der Fa. Nunc, (Roskilde, Dänemark) bevorzugt. Je Verdünnungsstufe wird eine Testplatte mit je 100 »l pro Vertiefung angelegt.
Die mit den Antigenlösungen gefüllten Testplatten werden 18 h bei 20.C belassen, dann werden die Lösungen in den Vertiefungen abgesaugt und die Näpfe dreimal mit Tris/Citronensäurelösung (0,05 M, pH 7,4), enthaltend 0,2% Rinderserumalbumin, durch Füllen und Absaugen gewaschen und die Testplatten anschliessend über Kieselgel bei 20°C getrocknet.

### 2. Herstellung eines Peroxidase-markierten Antikörpers gegen T.pallidum:

Als Ausgangsmaterial für den Konjugat-Antikörper dient ein Poolserum von Spendern mit positiver Syphilis-Serologie. Ein Mindesttiter im TPHA von > 1:10.000 und positive VDRL- und Cardiolipin-KBR-Titer werden gefordert. Im T.pallidum-Western-Blot zeigen solche Sera komplexe Muster mit einer Vielzahl von reaktiven Banden.
Das Serum wird gegen Puffer von 30 mM Na₂HPO₄/Na₂PO₄, pH 7,2, dialysiert und auf eine Säule, gefüllt mit DEAE-Cellulose DE 32 (Whatman) und equilibriert mit dem gleichen Puffer, gegeben. Die im Durchlauf erhaltene IgG-Fraktion wird gegen PBS dialysiert und auf 10 mg/ml eingestellt. Es wurde verifiziert, daß diese Methode zu keinem Verlust von bestimmten Antikörper-Spezifitäten und zu keinem signifikanten Titerrückgang führt. Die Konjugation der Peroxidase ist ausfuhrlich in der EP-A-0 265 851 beschrieben.

### 3. Qualitative Bestimmung von humanen Antikörpern gegen T.pallidum

Serum, Plasma oder Liquor (je 25 »l) wurden in Wells von Anti- T.pallidum-Testplatten gegeben und 1 h bei 37°C inkubiert. Die Platten waren dabei abgeklebt. Anschließend wurden pro Vertiefung 100 »l Anti-T.pallidum/POD zugegeben und die Testplatte eine weitere Stunde bei 37°C inkubiert. Der inhalt der Vertiefungen wurde durch Absaugen entfernt und die Wells viermal mit Waschpuffer gewaschen.
Es wurden in jede Vertiefung 100 »l TMB-Substratzubereitung gegeben, 30 min bei 20 - 22°C inkubiert und die Inkubation durch Zugabe von 100 »l 1 N Schwefelsaure beendet. Die OD 450 der gefarbten Lösung wurde gegen einen Leerwert von PBS gemessen.
Als Anti-T.pallidum-positiv wurden solche Proben eingestuft, die einen OD-Wert von weniger als 60% des Wertes des negativen Kontroll-serums zeigten, als Anti-T.pallidum-grenzwertige Proben, deren OD 450-Werte im Bereich von 60 - 70% der Negativ-Kontrolle lagen und als Anti-T.pallidum-negativ Proben, die eine OD 450 von über 70% der Negativ-Proben zeigten.
Fig. 4 zeigt die Häufigkeitsverteilung (Histogramm) Von 128 Syphilis-positiven Proben, von 80 Serumproben von Autoimmunpatienten, von 53 Proben von Borreliose-Patienten und von 71 Proben klinisch gesunder Blutspender.

Aus der Abbildung ist ersichtlich, daß das Positiv-Kollektiv von dem Negativ-Kollektiv deutlich getrennt ist, wobei je eines der Seren der Autoimmunpatienten und von Normalspendern in den positiven Bereich fällt. Andererseits konnten alle Proben, die in diesem Test positiv anzeigten, durch Vergleichstests in anderen Techniken bestätigt werden.

### 4. Quantitative Bestimmung von humanen Antikörpern gegen T.pallidum

Die Testdurchführung entspricht derjenigen, die in Beispiel 3 beschrieben wurde. Zunächst wurde ein hochtitriges Poolserum (TPHA-Titer > 1:10000) im negativen Serum in den Stufen 1:1, 1:4, 1:16, 1:64 und 1:256 verdünnt. Diese verdünnten Serumproben repräsentieren den zu erwartenden Titerbereich nativer Seren. Die Proben wurden nun jeweils in PBS, pH 7,2, in Faktor-3-Verdünnungsstufen titriert.
Im gleichen Testansatz wurde das Standardserum L3 über 8 Stufen titriert (Faktor 3) und außerdem (in Doppelbestimmung) in den Verdünnungsstufen 1:20 und 1:500 mitgeführt. Die Titrationskurven zeigten den in Fig. 1 dargestellten Kurven vergleichbare Verläufe.
Zunächst wurden die 50%-Titer der 5 verdünnten Serumproben entsprechend Fig. 1 bestimmt und auf den 50%-Titer des Standardserums L3 (Sollwert 40 IU) bezogen. Schließlich wurde der Gehalt (in IU) der verdünnten Serumproben bei jeder Verdünnungsstufe rechnerisch ermittelt (Anlage 1) und gegen die graphisch (nach der 50%-Methode) ermittelten Werte aufgetragen.

### 5. Nachweis von löslichem T.pallidum-Antigen

Bei diesem Testansatz wurde anstelle einer Serumprobe 60 »l einer Antigenlösung, entsprechend der Beschreibung in Abschnitt 4, in PBS verdünnt (ab 10⁹ Bakterienäquivalente pro ml,Verdünnungsfaktor 3) und mit der als optimal ermittelten Konjugatverdünnung (60 »l) in einer unbeschichteten Mikrotiterplatte für 1 h bei 37°C inkubiert. Nach Übertragung von 100 »l dieses Ansatzes in die Testplatte wurde für eine weitere Stunde bei 37°C inkubiert und nach 4-maligem Waschen wurde der Test durch Substratzugabe und Auswertung, wie unter Beispiel 3 beschrieben, ausgeführt. Fig. 5 zeigt, daß mit dieser Methode Keimzahlen von mehr als 10⁶ bis 10⁷ Bakterienäquivalenten pro ml, entsprechend 0,1 - 1 »g/ml nachgewiesen werden können.

### 6. Kompetitiver ELISA zum Nachweis von Serumantikörpern gegen Toxoplasma gondii:

Die Kavitäten einer Mikrotitrationsplatte werden mit je 100 »l einer Lösung von Toxoplasma gondii-Ultraschall-Antigen (vom RH-Stamm) in 0,01 M Bicarbonat-Puffer, pH 9,5 gefüllt und 20 h bei Raumtemperatur belassen. Das Waschen und Trocknen der Platten erfolgt wie in Beispiel 1 beschrieben.
Zur Gewinnung eines hochtitrigen Antiserums wurden Kaninchen nach Standardmethoden über 3 Wochen mit dem gleichen Antigen immunisiert. Die Methode der DE-Fraktionierung und der GMBS-Konjugation an Meerettich-Peroxidase ist in Beispiel 2 beschrieben.
Bei der Testdurchführung des kompetitiven ELISA werden zunächst je 25 »l der Serumprobe in die Testplatte vorgelegt und innerhalb von 15 min werden je 100 »l des Konjugatantikörpers in PBS zugefügt. Nach einer 1-stündigen Inkubation bei 37°C wird der Testansatz, wie in 3 beschrieben, 4-mal gewaschen. Nach Zugabe des Chromogens TMB wird der Test, wie beschrieben, durchgeführt und ausgewertet.

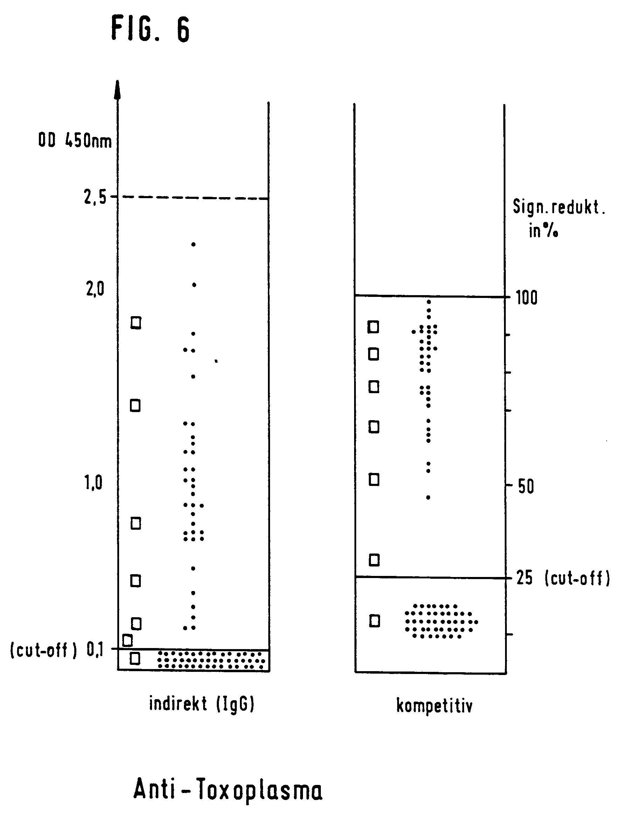

Zum Vergleich wurde ein Kollektiv von 80 Blutspendersera auch im indirekten IgG-ELISA, unter Verwendung eines Anti-Human-IgG/POD-Konjugates vom Kaninchen, in einer Serumverdünnung von 1:231 getestet (Enzygnost-Toxoplasmosis/IgG). Fig. 6 zeigt eine sehr gute Übereinstimmung der Methoden, wobei der kompetitive ELISA eine bessere Positiv/Negativ-Diskriminierung und eine technisch einfachere Durchführung bietet. Die vergleichbare Sensitivität ist aus dem Reaktionsausfall der Titrationsreihe eines positiven Standardserums (siehe Fig. 6) ersichtlich.

### 7. Kompetitiver ELISA zum Nachweis von Serumantikörpern gegen das Rubella-Virus:

Die Beschichtung der Mikrotestplatten und die Testdurchführung entspricht der in 6. beschriebenen Methodik.
Rubella-Virus (Stamm RA27/3) wurde in BHK-Zellen gezüchtet, aus dem Zellkulturüberstand mit Polyethylenglykol (PEG) 6000 präzipitiert und einer Tween® 80/Diethyläther-Behandlung nach Laufs und Thomssen (Arch.Ges.Virusforschung (1968) 24:164-180) unterzogen. Für die Plattenbeschichtung wurde das lösliche Antigen in PBS, pH 7,2, gelöst.
Für die Herstellung des Antikörper-POD-Konjugates wurde ein hochtitriger Serumpool von mehr als 5 Spendern (ELISA-IgG-Titer jeweils größer als 1:10000) nach der in EP 0 265 851 beschriebenen Methode fraktioniert und die reine IgG-Fraktion nach der GMBS-Methode an Meerettich-Peroxidase gekoppelt.
Bei der Auswahl der Sera wurde darauf geachtet, daß deren Reaktivität auf Kontrollantigen im indirekten IgG-ELISA möglichst niedrig war.
Ein Kollektiv von 90 Blutspendersera wurde vergleichend im indirekten IgG-ELISA (Serumverdünnung 1:231; Anti-Human-IgG vom Kaninchen) und im kompetitiven ELISA analog den Ausführungen in Beispiel 6. getestet.

### 8. Linearisierung des Verfahrens:

Auf einzelnen Riegeln einer jeden Antigenverdünnungsstufe wird zunächst eine Konjugattitration in Gegenwart eines Syphilis-negativen Spenderserums durchgeführt. Hierzu werden zunächst je 25 »l der Serumprobe für 1 h bei 37° C inkubiert und anschließend mit je 100 »l des Anti-T. pallidum/POD-konjugates in einer Verdünnungsreihe von 1:50, 100, 150, 200, 300, 400, 600 und 800, (in Tris-HCl, 1,3 M, pH 7,2) versetzt und eine weitere Stunde bei 37° C inkubiert.
Nach 4-maligem Waschen mit PBS, pH 7,2, enthaltend 0,1 % Tween® 20 wird anschließend in jede Vertiefung 100 »l TMB Substratzübereitung gegeben und 30 min bei 20 bis 22° C inkubiert. Die Inkubation wird beendet durch Zugabe von jeweils 100 »l 0 N Schwefelsäure. Die Extinktion der Lösungen wird gegen PBS bei 450 nm gemessen. Es werden diejenigen Antigen-/Konjugatkombinationen für weitere Optimierungsversuche ausgewahlt, welche Extinktionen (OD 450) zwischen 1,5 und 1,8 zeigten.
In einem weiteren Testansatz wurden bei jeweils aufeinander eingestellten Antigen-/Konjugatkonzentrationen Serumproben getestet, deren Gehalt an T.pallidum-spezifischen Antikörpern in konventionellen Tests bekannt war.
Es wird diejenige Antigen-/Konjugatkombination als geeignet ausgewählt, bei der die Signalreduktion linear mit dem Logarithmus des Titers korreliert. Optimale Ergebnisse werden z. B. bei einer Antigenverdünnung von 1:300 (entspricht etwa 0,7 »g/ ml) und einer Konjugatverdünnung von 1:200 erzielt. Da bei der Bestimmung von Anti-T.pallidum eine Grenzwertfestlegung von OD 450 > 70 % der Extinktion der Negativ-Kontrolle für Negative und von OD 450 < 70 % für Syphilis-positive als günstig angesehen wird, wird für die Herstellung von Microtestplatten zur Bestimmung von Anti-T.pallidum bei der Behandlung der Wells, die auch als Beschichtung bezeichnet wird, die Konzentration von ca. 1 »g/ml gewählt.

### Legende

Fig. 1: Für den kompetitiven Syphilis-ELISA typische Titrationskurven (Serumproben 1, 2 und 3 stellen reine IgM-Fraktionen, in negativem Serum aufgenommen, dar).

Fig. 2: Korrelation der Signalreduktion (in %) mit dem Logarithmus des 50%-Titers (für 48 Syphilis-positive Serumproben).

Fig. 3: Korrelation des kompetitiven ELISA (Enzygnost-Sy philis) mit dem TPHA (Cellognost-Syphilis, H).

Fig. 4: Häufigkeitsverteilung (Histogramm) von Syphilispositiven (< 60% Signal der Negativ-Kontrolle) und Syphilis-negativen (> 70% Signal der Negativ-Kontrolle) Serumproben über den gesamten Meßbereich.

Fig. 5: Korrelation der durch lineare Interpolation errechneten mit den graphisch ermittelten Titern (in IU). Dargestellt sind die Meßwerte für 5 unterschiedlich starke Serumproben in Abhängigkeit von der Probenverdünnung.

Fig. 6: Abhängigkeit der Signalreduktion im kompetitiven ELISA (modifizierte Version) von der Antigenkonzentration.

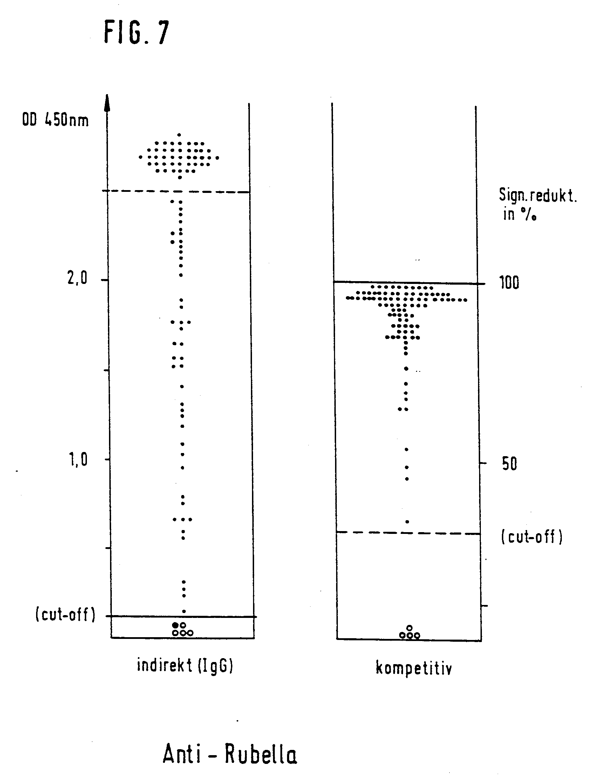

Fig. 7: Vergleich der Meßwerte des indirekten (IgG)-ELISA (Enzygnost-Toxo-plasmosis/IgG) und des kompetitiven Toxoplasma-Antikörper-ELISA (80 Sera gesunder, asymptomatischer Blutspender). Die Abbildung zeigt, daß der kompetitive ELISA eine deutlichere Positiv-/Negativ-Diskriminierung erlaubt als der indirekte ELISA.

## Patentansprüche (Patentansprüche für folgende(n) Vertragsstaat(en): AT, BE, CH, DE, FR, GB, IT, LI, LU, NL, SE)

1. Immunchemisches kompetitives Verfahren zum Nachweis und zur Bestimmung von Antikörpern gegen die Erreger von Syphilis, Toxoplasmose, Röteln, Cytomegalie, Amoebiasis, Echinokokkosis, Tetanus und Keuchhusten unter Verwendung einer Festphase bestehend aus einem Träger und daran irreversibel gebundenen Membranproteinen der jeweiligen Erreger und markierten Antikörpern, gerichtet gegen die wesentlichen Antigene des jeweiligen Erregers, wobei die Antikörper um die Bindungsstellen an der Festphase konkurrieren, dadurch gekennzeichnet, daß die Proteine irreversibel direkt oder über einen nicht immunchemisch bindenden Spacer an den Träger gebunden sind.

2. Verfahren nach Anspruch 1, dadurch gekennzeichnet, daß die Erreger die Erreger von Syphilis, Toxoplasmose und Röteln sind.

3. Verfahren nach Anspruch 1, dadurch gekennzeichnet, daß die Antigen-/Antikörperkonjugatkombination so eingestellt ist, daß die Signalreduktion linear mit dem Logarithmus des Titers korreliert.

4. Verfahren nach Anspruch 3, dadurch gekennzeichnet, daß die Auswertung rechnerisch, insbesondere mit Hilfe eines entsprechenden Computerprogrammes erfolgt.

5. Testbesteck zum Nachweis und zur Bestimmung von Antikörpern in einem immunchemischen kompetitiven Verfahren gemäß Anspruch 1, enthaltend einen Träger, an dem Membranproteine des jeweiligen Erregers direkt oder über einen nicht immunchemisch bindenden Spacer gebunden sind, markierte Antikörper gegen die genannten Membranproteine und gegebenenfalls Reagenzien zum Nachweis der Markierung.

6. Testbesteck nach Anspruch 5, dadurch gekennzeichnet, daß es aus einem Element besteht, in dem die Festphase und in trockener Form die für den Nachweis und die Bestimmung erforderlichen Reagenzien ganz oder teilweise enthalten sind.

7. Verwendung eines Testbestecks nach mindestens einem der Ansprüche 5 und 6 in einem Verfahren zum Nachweis und zur Bestimmung von Antikörpern gegen die Erreger von Syphilis, Toxoplasmose, Röteln, Cytomegalie, Amoebiasis, Echinokokkosis, Tetanus, und Keuchhusten.

8. Immunchemisches, kompetitives Verfahren zum Nachweis und zur Bestimmung von Erregern von Syphilis, Toxoplasmose, Röteln, Cytomegalie, Amoebiasis, Echinokokkosis, Tetanus und Keuchhusten unter Verwendung einer Festphase bestehend aus einem Träger und daran irreversibel gebundenen Antigenen der jeweiligen Erreger und markierten Antikörper, gerichtet gegen die wesentlichen Antigene des jeweiligen Erregers, wobei zunächst außerhalb des Testgefäßes die Probe mit den markierten Antikörpern vorinkubiert wird und dann zu der Festphase hinzugegeben wird, wobei die Antikörper um die Bindungsstelle an der Festphase konkurrieren, dadurch gekennzeichnet, daß die Proteine irreversibel direkt oder über einen nicht immunchemisch bindenden Spacer an den Träger gebunden sind.

## Patentansprüche (Patentansprüche für folgende(n) Vertragsstaat(en): ES)

1. Immunchemisches kompetitives Verfahren zum Nachweis und zur Bestimmung von Antikörpern gegen die Erreger von Syphilis, Toxoplasmose, Röteln, Cytomegalie, Amoebiasis, Echinokokkosis, Tetanus und Keuchhusten unter Verwendung einer Festphase bestehend aus einem Träger und daran irreversibel gebundenen Membranproteinen der jeweiligen Erreger und markierten Antikörpern, gerichtet gegen die wesentlichen Antigene des jeweiligen Erregers, wobei die Antikörper um die Bindungsstellen an der Festphase konkurrieren, dadurch gekennzeichnet, daß die Proteine irreversibel direkt oder über einen nicht immunchemisch bindenden Spacer an den Träger gebunden sind.

2. Verfahren nach Anspruch 1, dadurch gekennzeichnet, daß die Erreger die Erreger von Syphilis, Toxoplasmose und Röteln sind.

3. Verfahren nach Anspruch 1, dadurch gekennzeichnet, daß die Antigen-/Antikörperkonjugatkombination so eingestellt ist, daß die Signalreduktion linear mit dem Logarithmus des Titers korreliert.

4. Verfahren nach Anspruch 3, dadurch gekennzeichnet, daß die Auswertung rechnerisch, insbesondere mit Hilfe eines entsprechenden Computerprogrammes erfolgt.

5. Verfahren gemäß Anspruch 1, dadurch gekennzeichnet, daß ein Testelement verwendet wird, in dem die Festphase und in trockener Form die für den Nachweis und die Bestimmung erforderlichen Reagenzien ganz oder teilweise enthalten sind.

6. Immunchemisches, kompetitives Verfahren zum Nachweis und zur Bestimmung von Erregern von Syphilis, Toxoplasmose, Röteln, Cytomegalie, Amoebiasis, Echinokokkosis, Tetanus und Keuchhusten unter Verwendung einer Festphase bestehend aus einem Träger und daran irreversibel gebundenen Membranproteinen der jeweiligen Erreger und markierten Antikörper, gerichtet gegen die wesentlichen Antigene des jeweiligen Erregers, wobei zunächst außerhalb des Testgefäßes die Probe mit den markierten Antikörpern vorinkubiert wird und dann zu der Festphase hinzugegeben wird, wobei die Antikörper um die Bindungsstelle an der Festphase konkurrieren, dadurch gekennzeichnet, daß die Proteine irreversibel direkt oder über einen nicht immunchemisch bindenden Spacer an den Träger gebunden sind.

## Claims (Claims for the following Contracting State(s): AT, BE, CH, DE, FR, GB, IT, LI, LU, NL, SE)

1. An immunochemical competitive method for the detection and determination of antibodies against the organisms causing syphilis, toxoplasmosis, rubella, cytomegaly, amebiasis, echinococcosis, tetanus and whooping cough by use of a solid phase composed of a carrier and, irreversibly bound thereto, membrane proteins of the particular causative organisms and labeled antibodies directed against the essential antigens of the particular causative organism, there being competition between the antibodies for the binding sites on the solid phase, wherein the proteins are irreversibly bound to the carrier directly or via a non-immunochemically binding spacer.

2. The method as claimed in claim 1, wherein the causative organisms are the organisms causing syphilis, toxoplasmosis and rubella.

3. The method as claimed in claim 1, wherein the antigen/ antibody conjugate combination is adjusted so that the signal reduction is linearly related to the logarithm of the titer.

4. The method as claimed in claim 3, wherein the evaluation is by computation, in particular with the aid of an appropriate computer program.

5. A test kit for the detection and determination of antibodies in an immunochemical competitive method as claimed in claim 1, containing a carrier to which membrane proteins of the particular causative organism are bound directly or via a non-immunochemically binding spacer, labeled antibodies against the said membrane proteins and, where appropriate, reagents for detecting the labeling.

6. A test kit as claimed in claim 5, which is composed of an element in which the solid phase, and, in dry form, the reagents required for the detection and determination, in whole or in part are contained.

7. The use of a test kit as claimed in at least one of claims 5 and 6 in a method for the detection and determination of antibodies against the organisms causing syphilis, toxoplasmosis, rubella, cytomegaly, amebiasis, echinococcosis, tetanus and whooping cough.

8. An immunochemical competitive method for the detection and determination of organisms causing syphilis, toxoplasmosis, rubella, cytomegaly, amebiasis, echinococcosis, tetanus and whooping cough by use of a solid phase composed of a carrier and, irreversibly bound thereto, antigens of the particular causative organisms and labeled antibodies directed against the essential antigens of the particular causative organism, with the sample being initially preincubated with the labeled antibodies outside the test vessel and then added to the solid phase, there being competition between the antibodies for the binding site on the solid phase, wherein the proteins are irreversibly bound to the carrier directly or via a non-immunochemically binding spacer.

## Claims (Claims for the following Contracting State(s): ES)

1. An immunochemical competitive method for the detection and determination of antibodies against the organisms causing syphilis, toxoplasmosis, rubella, cytomegaly, amebiasis, echinococcosis, tetanus and whooping cough by use of a solid phase composed of a carrier and, irreversibly bound thereto, membrane proteins of the particular causative organisms and labeled antibodies directed against the essential antigens of the particular causative organism, there being competition between the antibodies for the binding sites on the solid phase, wherein the proteins are irreversibly bound to the carrier directly or via a non-immunochemically binding spacer.

2. The method as claimed in claim 1, wherein the causative organisms are the organisms causing syphilis, toxoplasmosis and rubella.

3. The method as claimed in claim 1, wherein the antigen/antibody conjugate combination is adjusted so that the signal reduction is linearly related to the logarithm of the titer.

4. The method as claimed in claim 3, wherein the evaluation is by computation, in particular with the aid of an appropriate computer program.

5. The method as claimed in claim 1, wherein a test element is used in which the solid phase, and, in dry form, the reagents required for the detection and determination, in whole or in part are contained.

6. An immunochemical competitive method for the detection and determination of antibodies against the organisms causing syphilis, toxoplasmosis, rubella, cytomegaly, amebiasis, echinococcosis, tetanus and whooping cough by use of a solid phase composed of a carrier and, irreversibly bound thereto, membrane proteins of the particular causative organisms and labeled antibodies directed against the essential antigens of the particular causative organism, with the sample being initially preincubated with the labeled antibodies outside the test vessel and then added to the solid phase, there being competition between the antibodies for the binding site on the solid phase, wherein the proteins are irreversibly bound to the carrier directly or via a non-immunochemically binding spacer.

## Revendications (Revendications pour l'(les) Etat(s) contractant(s) suivant(s): AT, BE, CH, DE, FR, GB, IT, LI, LU, NL, SE)

1. Procédé immunochimique compétitif pour la détection et pour la détermination d'anticorps dirigés contre les agents pathogènes de la syphilis, la toxoplasmose, la rubéole, la maladie des inclusions cytomégaliques, l'amibiase, l'échinococcose, le tétanos et la coqueluche, au moyen d'une phase solide constituée d'un support et de protéines membranaires des agents pathogènes respectifs liées irréversiblement à celui-ci, et d'anticorps marqués, dirigés contre les antigènes essentiels de l'agent pathogène respectif, les anticorps entrant en compétition pour les sites de liaison sur la phase solide, caractérisé en ce que les protéines sont irréversiblement liées au support directement ou par l'intermédiaire d'un espaceur non susceptible de liaison immunochimique.

2. Procédé selon la revendication 1, caractérisé en ce que les agents pathogènes sont les agents pathogènes de la syphilis, de la toxoplasmose et de la rubéole.

3. Procédé selon la revendication 1, caractérisé en ce que la combinaison antigène/anticorps est ajustée de manière que la réduction du signal soit en correlation linéaire avec le logarithme du titre.

4. Procédé selon la revendication 3, caractérisé en ce que l'évaluation s'effectue par le calcul, en particulier à l'aide d'un programme d'ordinateur approprié.

5. Nécessaire d'essai pour la détection et pour la détermination d'anticorps dans un procédé immunochimique compétitif selon la revendication 1, contenant un support auquel des protéines membranaires de l'agent pathogène respectif sont liées directement ou par l'intermédiaire d'un espaceur non susceptible de liaison immunochimique, des anticorps marqués dirigés contre lesdites protéines membranaires, et éventuellement des réactifs pour la détection du marquage.

6. Nécessaire d'essai selon la revendication 5, caractérisé en ce qu'il consiste en un élément dans lequel sont contenus en partie ou en totalité la phase solide et, sous forme sèche, les réactifs requis pour la détection et la détermination.

7. Utilisation d'un nécessaire d'essai selon au moins l'une des revendications 5 et 6, dans un procédé pour la détection et la détermination d'anticorps dirigés contre les agents pathogènes de la syphilis, la toxoplasmose, la rubéole, la maladie des inclusions cytomégaliques, l'amibiase, l'échinococcose, le tétanos et la coqueluche.

8. Procédé immunochimique compétitif pour la détection et pour la détermination d'agents pathogènes de la syphilis, la toxoplamose, la rubéole, la maladie des inclusions cytomégaliques, l'amibiase, l'échinococcose, le tétanos et la coqueluche, au moyen d'une phase solide constituée d'un support et d'antigènes des agents pathogènes respectifs, irréversiblement liés à celui-ci, et d'anticorps marqués, dirigés contre les antigènes essentiels de l'agent pathogène respectif, dans lequel en premier lieu on met l'échantillon, à l'extérieur du récipient d'essai, à préincuber avec les anticorps marqués, et ensuite on l'ajoute à la phase solide, ce par quoi les anticorps entrent en compétition pour les sites de liaison sur la phase solide, caractérisé en ce que les protéines sont irréversiblement liées au support, directement ou par l'intermédiaire d'un espaceur non susceptible de liaison immunochimique.

## Revendications (Revendications pour l'(les) Etat(s) contractant(s) suivant(s): ES)

1. Procédé immunochimique compétitif pour la détection et pour la détermination d'anticorps dirigés contre les agents pathogènes de la syphilis, la toxoplasmose, la rubéole, la maladie des inclusions cytomégaliques, l'amibiase, l'échinococcose, le tétanos et la coqueluche, au moyen d'une phase solide constituée d'un support et de protéines membranaires des agents pathogènes respectifs liées irréversiblement à celui-ci, et d'anticorps marqués, dirigés contre les antigènes essentiels de l'agent pathogène respectif, les anticorps entrant en compétition pour les sites de liaison sur la phase solide, caractérisé en ce que les protéines sont irréversiblement liées au support directement ou par l'intermédiaire d'un espaceur non susceptible de liaison immunochimique.

2. Procédé selon la revendication 1, caractérisé en ce que les agents pathogènes sont les agents pathogènes de la syphilis, de la toxoplasmose et de la rubéole.

3. Procédé selon la revendication 1, caractérisé en ce que la combinaison antigène/anticorps est ajustée de manière que la réduction du signal soit en correlation linéaire avec le logarithme du titre.

4. Procédé selon la revendication 3, caractérisé en ce que l'évaluation s'effectue par le calcul, en particulier à l'aide d'un programme d'ordinateur approprié.

5. Procédé selon la revendication 1, caractérisé en ce que l'on utilise un élément d'essai dans lequel sont contenus en partie ou en totalité la phase solide et, sous forme sèche, les réactifs requis pour la détection et la détermination.

6. Procédé immunochimique compétitif pour la détection et pour la détermination d'agents pathogènes de la syphilis, la toxoplamose, la rubéole, la maladie des inclusions cytomégaliques, l'amibiase, l'échinococcose, le tétanos et la coqueluche, au moyen d'une phase solide constituée d'un support et de protéines membranaires des agents pathogènes respectifs irréversiblement liées à celui-ci, et d'anticorps marqués, dirigés contre les antigènes essentiels de l'agent pathogène respectif, dans lequel en premier lieu on met l'échantillon, à l'extérieur du récipient d'essai, à pré-incuber avec les anticorps marqués, et ensuite on l'ajoute à la phase solide, ce par quoi les anticorps entrent en compétition pour les sites de liaison sur la phase solide, caractérisé en ce que les protéines sont irréversiblement liées au support, directement ou par l'intermédiaire d'un espaceur non susceptible de liaison immunochimique.
